# EUROPEAN PATENT SPECIFICATION

(11) **EP 0 511 690 B1**
(45) Date of publication and mention of the grant of the patent: **30.07.1997**
(21) Application number: 92111024.3
(22) Date of filing: 24.03.1987
(51) Int. Cl.: C12P 19/06, C12R 1/64

(54) **Family of xanthan-based polysaccharide polymers including non-acetylated and/or non-pyruvylated gum**
Familie von auf Xanthan basierenden Polysaccharidpolymeren, welche Nichtacetylierten und/oder Nichtpyruvylierten Gum beeinhalten
Famille de polymères polysaccharides à base de xanthane comprenant une gomme non-acétylée et/ou non-pyruvylée

(30) Priority: 24.03.1986 US 842945; 26.03.1986 US 844435; 23.03.1987 US 29090
(43) Date of publication of application: 04.11.1992
(62) Divisional of application: 87902919.7
(73) Proprietor: GETTY SCIENTIFIC DEVELOPMENT COMPANY, Houston Texas 77215-0070 (US)
(72) Inventor: Doherty, Daniel H., Boulder, CO 80303 (US); Ferber, Donna M., Boulder, CO 80303 (US); Marrelli, John D., Houston, TX 77009 (US); Vanderslice, Rebecca W., Boulder, CO 80303 (US)
(74) Representative: Grünecker, Kinkeldey, Stockmair & Schwanhäusser Anwaltssozietät

(56) References cited:
- WO-A-87/05938
- MACROMOLECULES, vol. 16, no. 5, 1983, US; M. RINAUDO et al., pp. 816-819/
- CARBOHYDRATE POLYMERS, vol. 3, no. 1, 1983, Barking (GB); I.J. BRADSHAW et al., pp. 23-38/
- CHEMICAL ABSTRACTS, vol. 103, no. 18, 1985, Columbus, OH (US); J.C. PHILIPS et al., p. 135, no. 144288t/
- CHEMICAL ABSTRACTS, vol. 60, no. 8, 1964, Columbus, OH (US); D.G. ORENTASI et al., no. 9623g/
- CARBOHYDRATE POLYMERS, vol. 1, no. 2, 1981, Barking (GB); I.W. SUTHERLAND, pp. 107-115/

## Description

### BACKGROUND OF THE INVENTION

This invention relates to polysaccharide polymers. In particular, it relates to xanthan-based polysaccharide polymers, defined here as polymers structurally similar to xanthan gum and produced by components of the xanthan biosynthetic pathway, including non-acetylated, non-pyruvylated, non-acetylated and non-pyruvylated, low pyruvylated and fully pyruvylated xanthan (pentamer) gums.

Xanthan gum is produced by bacteria of the genus Xanthomonas, in particular by microorganisms of the species X. campestris. Xanthan gum is a widely used product due to its unusual physical properties, i.e., its extremely high specific viscosity and its pseudoplasticity. It is commonly used in foods as a thickening agent and in secondary or tertiary oil recovery as a mobility control and profile modification agent, as well as in petroleum drilling fluids.

Chemically, xanthan gum is an anionic heteropolysaccharide. The repeating unit of the polymer is a pentamer composed of five sugar moieties, specifically two glucose, one glucuronic acid and two mannose moieties. These sugar residues are arranged such that the glucose moieties form the backbone of the polymer chain, with side chains of mannose-glucuronic acid-mannose residues generally extending from alternate glucose moieties. Usually, this basic structure is specifically acetylated and pyruvylated, as described, for example, by Janson, P.E., Kenne, L., and Lindberg, B., in Carbohydrate Research, 45:275-282 (1975) and Melton, L.D., Minot, L., Rees, D.A., and Sanderson, G.R., in Carbohydrate Research, 46:245-257 (1976), each of which is specifically incorporated herein by reference. The extent of acetylation and pyruvylation is known to vary. The structure of xanthan gum is depicted below:

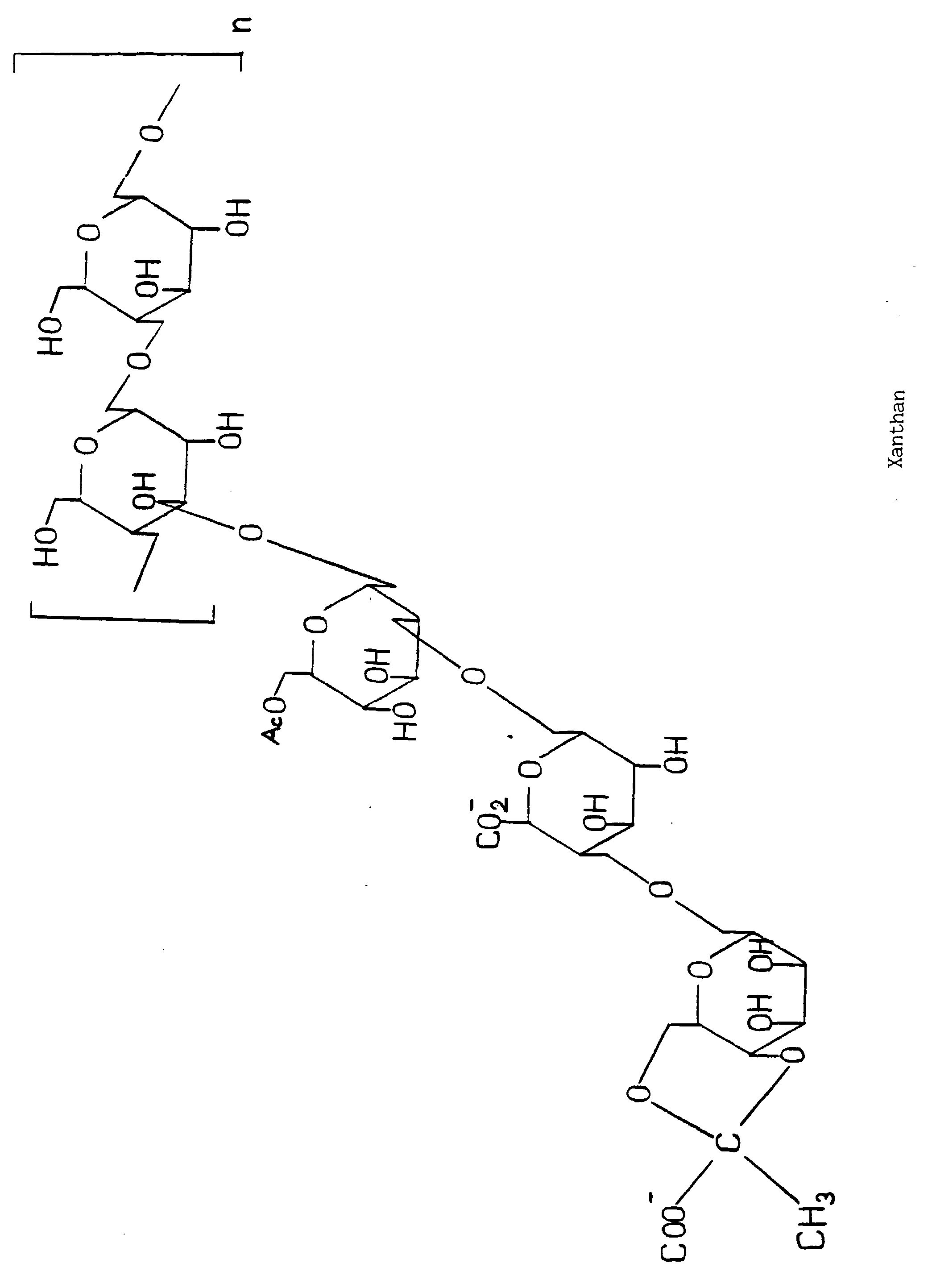

In spite of the broad utility of naturally-occurring xanthan gum, there are some situations where its physical properties become limiting. In particular, in secondary or tertiary oil recovery it is not uncommon for the temperature of the oil bearing reservoir and the salt concentrations in the reservoir brine to be higher than are optimal for xanthan solutions. When these conditions occur, xanthan can precipitate, flocculate and/or lose its viscosity. Therefore, new viscosifying products which perform well at various conditions encountered during oil recovery, such as high temperature and high salt concentrations would be desirable.

The present invention discloses a family of xanthan-based polysaccharides having improved properties relative to naturally-occurring xanthan gum. Modifications of xanthan gum have been previously described. For example, Bradshaw et al. (Carbohydrate Polymers, 3:23-38 (1983)) describe methods for preparing chemically-modified xanthan gum which are deacetylated or depyruvylated. Various means of chemically deacetylating xanthan gum produced by Xanthomonas campestris also are described in U.S. Patent Nos. 3,000,790 and 3,054,689. To date, the sole method utilized for these deacetylation processes has been through chemical removal of the acetate moieties from normally acetylated xanthan gum. It has been found that chemical processes for deacetylating xanthan gums result in a number of undesirable side effects and may cause hydrolysis of the glycosidic backbone, resulting in an irreversible change in the conformation of the molecule and lowered molecular weight.

Some of the rheological properties of deacetylated xanthan in aqueous media are known. See, e.g., Tako and Nakamura, Agric. Biol. Chem. 48:2987-2993 (1984) and U.S. Patents Nos. 3,000,790 and 3,054,689. Also, a method of increasing the viscosity of an aqueous solution using a deacetylated polysaccharide is described in U.S. Patent No. 3,096,293. Thus, a method for obtaining non-acetylated xanthan which does not cause untoward side effects has been sought.

Xanthan gum can be chemically depyruvylated as well, as described by Holzwarth and Ogletree in Carbo. Res. 76:277-280 (1979). This chemical method of depyruvylation also can alter the xanthan polymeric unit and/or cause hydrolysis of the glycosidic backbone. While a strain of X. campestris has been described in U.S. Patent No. 4,296,203 which produces non-pyruvylated xanthan gum, this non-pyruvylated gum was either fully acetylated or deactylated using chemical means. The present inventors believe that a xanthan polysaccharide that is both non-acetylated and non-pyruvylated will have improved rheological and viscosifying properties and have therefore sought such a gum along with a microbial method of manufacturing it.

Additionally, the extent of acetylation of the internal mannose on the xanthan side chain and the extent of the pyruvylation of the terminal mannose may vary. The present inventors believe that a fully acetylated and/or fully pyruvylated xanthan will have improved rheological properties for certain oil recovery purposes.

### SUMMARY OF THE INVENTION

An object of the present invention is to provide a family of polysaccharide polymers which are better viscosifiers of water than naturally-occurring xanthan gum. Another object of the present invention is to provide a family of polysaccharide polymers having improved rheological properties over naturally-occurring xanthan gum at elevated temperatures and/or in the presence of salts and which members possess other desired properties.

It is also an object of the present invention to provide an in vitro method for obtaining certain of these products and microorganisms having the ability to produce members of this family of polysaccharide polymers in vivo. A further object of the present invention is to provide processes for preparing members of this family of polysaccharides by aerobically fermenting microorganisms having the ability to produce the various polysaccharide polymers.

Additional objects and advantages of the invention will be set forth in part in the description which follows, and in part will be obvious from the description, or may be learned by practice of the invention. The objects and advantages may be realized and attained by means of the instrumentalities and combinations particularly pointed out in the appended claims.

To further achieve the objects and in accordance with the purposes of the present invention, there is provided a composition comprising xanthan gum wherein the mannose moieties are not acetylated. This gum is hereinafter referred to as "non-acetylated xanthan." A second structure is referred to herein where the terminal mannose moieties of xanthan gum are not pyruvylated. This gum will be referred to as "non-pyruvylated xanthan." Moreover, there has been disclosed a gum which is neither acetylated nor pyruvylated. This polymer is hereinafter referred to as "non-acetylated, non-pyruvylated xanthan gum." Also, the present invention relates to a xanthan gum herein referred to as "fully acetylated xanthan gum," wherein at least 90% of the internal mannose moieties are acetylated, preferably 95% and more preferably 100%, are acetylated. Also, the present invention relates to a xanthan gum wherein at least 90% of the terminal mannose moieties, preferably 95% and more preferably 100%, are pyruvylated, herein referred to as "fully-pyruvylated xanthan gum."

This invention also contemplates processes for the production of the polysaccharide polymers described above. The polysaccharide polymers of this invention can be made generally by genetic manipulations of the microbial biosynthetic pathways which lead to the production of polysaccharides. In particular, microbial pathways for the production of xanthan gum may be manipulated to create an in vivo or in vitro system for the production of an altered polymeric unit. Thus, systems can be created through the use of regulatable Acetylase and Ketalase genes, in particular, to create polysaccharides which are acetylated or pyruvylated to varying degrees. For example, it is contemplated that xanthan gum which is 10%, 20%, 30%, 40%, or 50% acetylated can be synthesized as well as xanthan which is 10%, 20%, 30%, 40%, 50%, 60%, 70%, or 80% pyruvylated. Microorganisms which produce the present polysaccharide polymers in vivo and methods of using these polysaccharide polymers are also disclosed.

Various strains of X. campestis described more fully hereinbelow have been deposited with the American Type Culture Collection, Rockville, Maryland, on March 21, 1986. These strains are X921, X1006, X934 and X1231 and have been deposited under Acccession Nos. 53472, 53473, 53474 and 67344, respectively.

It is understood that both the foregoing general description and the following detailed description are exemplary and explanatory only and are not restrictive of the invention, as claimed. The accompanying drawings, which are incorporated in and constitute a part of this specification, illustrate various embodiments of the invention and, together with the description, serve to explain the principles of the invention.

### BRIEF DESCRIPTION OF THE DRAWINGS

FIGURE 1 depicts the presumed pathway of xanthan gum biosynthesis. Abbreviations used are: Glu=Glucose; GluA=Glucuronic acid; Man=Mannose; Glu-Glu=Cellobiose; P=Phosphate; PP=Pyrophosphate; C55=Isoprenoid Lipid Carrier; PEP=Phosphoenolpyruvate; AcCoA=Acetylcoenzyme A; I-V=Glycosyltransferases; UDP=Uridine 5'-diphosphate; and GDP=Guanosine 5'-Diphosphate.

FIGURE 2 depicts a viscosity comparison between wild-type and non-pyruvylated gums.

FIGURE 3 depicts a viscosity comparison between non-acetylated and chemically-deacetylated gums.

FIGURE 4 depicts the approximate physical location of 3 TnK12 insertion mutations within the cloned gum gene cluster DNA of recombinant plasmid pRK290-H336. This figure also shows the approximate locations of SpeI restriction endonuclease cleavage sites in pRK290-H336.

### DETAILED DESCRIPTION OF THE INVENTION

Reference will now be made in detail to the presently preferred embodiments of the present invention which, together with the following examples, serve to explain the principles of the invention.

The polysaccharide polymers of the present invention have been described in detail above. These polysaccharide polymers can be produced in vitro with a cell-free enzyme system or can be produced in vivo by growing cells of an appropriate mutant strain. Other means of preparing the polysaccharide polymers are also described below.

### In Vitro Polysaccharide Synthesis

The basic method relating to the use of a cell-free systems to make non-variant xanthan gum is described by Ielpi, L., Couso, R.O., and Dankert, M.A. in FEBS Letters 130:253-256 (1981), specifically incorporated herein by reference. It has been found that a modified version of this method may be employed to create the variant polysaccharides of this invention.

For this novel, modified method, the in vitro cell-free system is prepared generally by lysing cells of a microorganism of the genus Xanthomonas, preferably Xanthomonas campestris, in the presence of a suitable buffer, preferably including EDTA, and obtaining the appropriate biosynthetic enzymes which are able to subsequently process exogenously added substrates. Alternate means of lysis may be used, including but not limited to sonication, French Pressure cell, detergent treatment enzyme treatment and combinations thereof.

Generally, to produce the variant polysaccharides of the present invention, a lysate of a microorganism possessing the enzymes required to assemble the desired polysaccharide is incubated with the appropriate substrates, which, depending on the gum desired, may include UDP-glucose, GDP-mannose, UDP-glucuronic acid, acetyl-CoA and phosphoenolpyruvate. The choice of substrates is dependent on the polysaccharide which it is desired to produce. For example, a non-acetylated polysaccharide is obtained by eliminating acetyl-CoA as a substrate. Similarly, a non-pyruvylated gum is obtained by eliminating phosphoenolpyruvate as a substrate. Chemical and/or enzymatic treatment of the cell lysates in order to deplete endogeneous substrates will be evident to one skilled in the art.

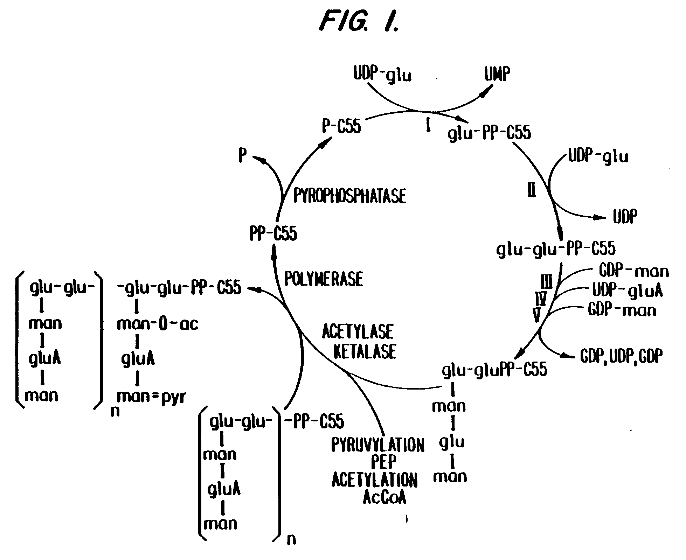

In addition, cell-free systems may be created from mutant organisms deficient in one or more of the enzymes of the xanthan biosynthetic pathway set forth in Figure 1. Such mutant-derived cell lysates would produce the variant gums described herein, either due solely to the mutation or due to the mutation in combination with a withheld substrate.

The biosynthetic process may, in one embodiment, be monitored by the incorporation of radiolabeled substrates into the polymeric units. Other methods may also be used to allow identification of the biosynthetic intermediates that are known to those of ordinary skill in the art. In particular, chromatographic methods have been developed to separate and to identify the oligosaccharide intermediates. These include thin layer chromatography and high-performance liquid chromatography.

The cell-free biosynthesis of xanthan has been found to be a time-dependent, sequential process that is dependent on the addition of all three specific sugar nucleotides. The background of non-specific incorporation of labeled substrate is minimal and does not interfere with the detection of the xanthan-specific polymer in the gum fraction.

The involvement of lipid carriers, specifically isoprenoid pyrophosphate, has been shown in several polysaccharide biosynthetic pathways. Additionally, the involvement of pyrophosphoryl-linked lipid carrier in xanthan biosynthesis has been demonstrated. Thus, the xanthan biosynthetic intermediates have been found to be recoverable in the organic soluble fraction with these carrier lipids. The recovered oligosaccharide can subsequently be freed from the carrier lipid by mild acid hydrolysis, for example, pH 2 for 20 minutes at 90°C and dephosphorylated with alkaline phosphatase for analysis.

Using these methods for recovery of intermediate products, it has been discovered that, under in vitro conditions, certain lysates of X. campestris mutants will produce non-acetylated or non-pyruvylated xanthan gum even in the presence of all substrates required for non-variant gum synthesis. In light of the teachings herein, these methods will enable one skilled in the art to identify cell lysates which produce other altered polysaccharides.

### In Vivo Polysaccharide Synthesis

The development of the cell-free synthesis process for the polysaccharides described above demonstrated that various Xanthomonas campestris cells have all the enzymes necessary to synthesize acetylated or non-acetylated polytetramer, non-acetylated and/or non-pyruvylated xanthan gum and fully-acetylated xanthan gum.

Furthermore, for whole cells to synthesize non-acetylated xanthan gum, a means of blocking the acetylation step during xanthan gum synthesis would be required. Additionally, for the whole cells to synthesize non-acetylated, non-pyruvylated xanthan gum, a means of blocking xanthan gum synthesis at both the acetylation and pyruvylation steps would be required. In one embodiment of the present invention, mutagenesis was employed to alter some of the genes responsible for these various reactions.

Transposons, including but not limited to Tn10 and Tn903, can be used to mutagenize Xanthomonas campestris. These transposons, in one embodiment, confer resistance to tetracycline and kanamycin, respectively. Transposons have the ability to insert themselves into genes wherein they cause mutations by interrupting the coding sequence. The transposons can be introduced into Xanthomonas campestris on various vectors, including on so-called suicide vectors, such as pRK2013. Vector pRK2013, as described by Ditta, G., Corbin, D. and Helinski, D.R. in Proc. Natl. Acad. Sci. U.S.A., 77:7347-7351 (1980), specifically incorporated herein by reference, has the ability to transfer itself into non-enteric bacteria, such as Xanthomonas campestris, but cannot replicate in that host. Thus, if the suicide vector is introduced into a population of Xanthomonas campestris cells and that population is subsequently challenged with either tetracycline or kanamycin, the individuals which survive are those in which one of the transposons has inserted into the genome of Xanthonomas campestris. Survivors of such a challenge can be screened for those which have lost the ability, to make xanthan gum. Such mutants may appear less mucoid than wild-type Xanthomonas campestris.

In other embodiments of the invention, other means of mutagenesis can be employed to generate mutants which have lost the abilitY to make xanthan gum or that do not acetylate and/or pyruvylate the gums they produce. Such means will readily occur to one skilled in the art, and include, without limitation, irradiation, recombinant DNA technology (in particular, as described in United States Patent Application Serial No. 842,944 of Capage et al., entitled "Recombinant-DNA Mediated Production of Xanthan Gum," filed March 26, 1986, and incorporated specifically herein by reference, and in Example 1 below) and chemical mutagen treatment. Examples of such mutagenesis procedures have been described by Miller, J.H. in Experiments in Molecular Genetics (1972); Davis, R.W., Bostein, D. and Roth, J.R. in Advanced Bacterial Genetics (1980); and Maniatis, T., Fritsch, E.F. and Sambrook, J. in Molecular Cloning (1982), Cold Spring Harbor.

Although mutants can first be chosen which appear less mucoid than wild-type organisms, those desired generally retain the ability to make some polysaccharide. Cell-free extracts of each of the xanthan mutants can be prepared and tested as noted above by the addition of different combinations of substrates and analysis of the resultant products.

Alternatively, appropriate mutants can be detected by assaying the culture broth of each mutant for the presence of the desired polysaccharide, e.g.xanthan gum which is non-acetylated. non-acetylated and non-pyruvylated, low pyruvate or fully-acetylated. Thus, mutants can be found which appear to be blocked at various positions of the xanthan gum pathway. A mutant of Xanthomonas campestris which produces non-acetylated xanthan gum (X1006), a mutant which produces non-pyruvylated gum (X921), a mutant which produces xanthan gum containing low levels of pyruvylation (less than 5% of the terminal mannose X934), and a mutant which produces non-acetylated, non-pyruvylated xanthan gum (X1231 (p41KS22)) have each been placed on deposit at the American Type Culture Collection, Rockville, Maryland, under Accession Nos. 53473, 53472, 53474 and 67344, respectively.

It is not beyond the scope of the invention to employ enzyme inhibitors of wild-type Acetylase and Ketalase to arrive at the same products. Still other alternatives for producing this family of polysaccharides are contemplated, including enzymatic and chemical degradation of natural xanthan gum.

The mutants can be grown under conditions generally known in the art for growth of wild-type Xanthomonas campestris. For example, they can be grown on suitable assimilable carbon sources such as glucose, sucrose, maltose, starch, invert sugar, complex carbohydrates such as molasses or corn syrup, various organic acids and the like. Mixtures of carbon sources can also be employed. The concentration of carbon source supplied is often between 10 and 60 grams per liter. Also necessary for growth are an assimilable source of organic or inorganic nitrogen, generally between about 0.1 and 10.0 grams per liter, and minerals, the choice of which are easily within the skill of the art. Examples of suitable nitrogen sources are ammonium salts, nitrate, urea, yeast extract, peptone, or other hydrolyzed proteinaceous materials or mixtures thereof. Examples of suitable minerals include phosphorus, sulfur, potassium, sodium, iron, magnesium; these are often added with a chelating agent such as EDTA or citric acid.

Optimal temperatures for growth of Xanthomonas campestris generally are between 18°C and 35°C, preferably between about 27°C and 30°C. Xanthomonas campestris cells are grown aerobically by supplying air or oxygen so that an adequate level of dissolved oxygen is maintained, for example, above about 10% of saturation. Preferably, the level is kept above about 20%. The pH often is maintained at about 6.0 to 8.0, preferably at about 6.5 to 7.5.

The polysaccharides of the present invention can be recovered from fermentation broths by a suitable means. Precipitation with isopropanol, ethanol, or other suitable alcohol readily yields the polysaccharides of this invention. Generally, alcohols are added to a concentration of about 50 to 75%, on the basis of volume, preferably in the presence of potassium chloride, sodium chloride or other salt. Alternatively, the polymers can be recovered from the broth by ultra-filtration.

Non-pyruvylated xanthan gums are superior to xanthan as viscosifiers of aqueous mediums in particular applications. The viscosity of solutions of non-acetylated xanthan and non-pyruvylated xanthan are retained at conditions of high temperature and/or high salinity. The products of this invention are thus ideally suited for use in secondary and tertiary oil recovery.

Mobility control solutions for use in enhanced oil recovery may also be prepared from the variant polysaccharide polymers disclosed herein. Solutions of the polysaccharide polymers at concentrations of from about 50 to about 3000 ppm are appropriate for such mobility control solutions. Other known additives may also be used in combination with these solutions to further enhance oil recovery. Such additives include, for example, surfactants, alkaline agents or metal or organic crosslinking agents.

The polysaccharide polymers, like xanthan gum, can also be used as thickening agents in foods, cosmetics, medicinal formulations, paper sizings, drilling muds, printing inks, and the like and as a gelling agent. In addition, they can be used to reduce frictional drag of fluid flow in pipes.

### EXAMPLE 1

This example shows the methods of mutagenesis and screening employed to generate X. campestris mutant strains having defects in Acetylase or Ketalase activity.

The genes encoding the enzymes of xanthan gum biosynthesis have been shown to comprise a set of clustered genes on the X. campestris chromosome. This "gum gene cluster" has been described in detail by Capage et al. Segments of gum gene DNA have been cloned on plasmid vectors such as pMW79 as detailed in Capage et al.

Regionally-directed mutagenesis was performed upon subcloned portions of the gum DNA carried in plasmid pMW79. These cloned DNA segments were mutagenized in vivo with transposons and in vitro, by using recombinant DNA technology to generate insertion, deletion, and substitution mutations within the cloned X. campestris DNA. In order to study the phenotypes conferred by these mutations, the plasmids carrying the mutations were transferred back into X. campestris and subsequently recombinants were identified in which the plasmid-borne, mutated gene had been inserted in the chromosome via homologous recombination. The tetracycline resistance encoded by Tn10 affords a convenient selective system for movement of mutations from a plasmid into the chromosome.

One such mutant strain (X1006) carried a Tn10 insertion that was found to cause inactivation of the Acetylase activity. This mutant strain was characterized as described in Example 2 and 3, and found to produce a polysaccharide that was non-acetylated. A second mutant strain was constructed by the in vitro insertion of a fragment of DNA containing the tetracycline resistance gene of Tn10 into a restriction site within the gum gene cluster. This mutant strain (X921) was found to be defective in the Ketalase activity. As found by the methods of Examples 2 and 3, this mutant produced xanthan that was non-pyruvylated.

A third mutant strain (X934) was also found that greatly reduces the ketalase activity. This mutant strain produces xanthan gum that has a very low level of pyruvylation: 1-5% of the level of pyruvylation found in normal xanthan.

The mutant strain X934 was found as described below. In preliminary experiments designed to study recombination between plasmid-borne X. campestris DNA and the X. campestris chromosome, the plasmid pTX655 was used as a model system. This plasmid carries a Tn10 insert in the middle of a 2.3 kb X. campestris PstI fragment cloned in plasmid RSF1010. This insertion of Tn10 causes the Gum- defect in the mutant strain X655 as described by Capage et al. and Vanderslice et al. The experiment was to mobilize pTX655 with plasmid pRK2013 and transfer it from E. coli into X. campestris by selecting for transfer of the tetracycline resistance encoded by Tn10. The initial results of this mating were anomalous and suggested that Tn10 did not express tetracycline resistance efficiently in X. campestris when carried on the plasmid, but that the drug resistance was more efficiently expressed when Tn10 was carried in the chromosome of X. campestris. This phenomenon has also been described for Tn10 in E. coli. There, it has been shown that strains carrying one copy of Tn10 inserted in the chromosome are resistant to significantly higher concentrations of tetracycline than are strains carrying Tn10 on a multicopy plasmid. The selection of Tet^{r} X. campestris out of the above mating resulted in a high frequency (0.5 per recipient) of progeny which grew very poorly (i.e., only small, watery colonies) on tetracycline. After prolonged incubation, a large fraction of the colonies (25%) produced sectors of more vigorously growing cells. More than 50% of these sectors appeared to be Gum- in morphology. These probably result from recombination between the plasmid-borne DNA containing the Tn10 insertion and the chromosomal wild type DNA. When the Tn10 is recombined into the chromosome, high-level Tet^{r} is obtained and the vigorously growing sector is observed. When these Gum-, Tet^{r} sectors were picked and restreaked on tetracycline, they grew well and displayed a characteristic Gum-morphology.

Gum⁺, Tet^{r} isolates were also characterized. Some of these strains (in particular X934) were found to contain the entire plasmid pTX655 inserted into the chromosome of X. campestris via homologous recombination. The chromosomal structure of the X934 strain was determined by Southern blot hybridization of the chromosomal DNA which shows that the plasmid sequences exist in a chromosomally integrated form.

### EXAMPLE 2

This example shows how the altered polysaccharides of the present invention can be prepared in vitro. For instance, it shows how non-acetylated and/or non-pyruvylated xanthan gum was prepared in vitro.

### Preparation of Lysates

Xanthomonas campestris B1459 S4-L or S4-L mutants described in Examples 1 and 5 were grown in YM (yeast-malt medium) supplemented with 2% (w/v) glucose as described by Jeanes, A. et al. in U.S. Department of Agriculture, ARS-NC-51, pp. 14 (1976), specifically incorporated herein by reference. Cultures were grown to late log phase at 30°C. The cells were harvested by centrifugation and washed with cold Tris-HCl,70mM, pH 8.2 with 10mM EDTA and were freeze-thawed three times by a procedure similar to Garcia, R.C. et al. described in European Journal of Biochemistry 43:93-105 (1974), specifically incorporated herein by reference. This procedure ruptured the cells, as was evidenced by the increased viscosity of the suspensions and the complete loss of cell viability (one of 10⁶ survivors) after this treatment. The freeze-thawed lysates were frozen in aliquots at -80°C. Protein concentration was determined with BIO RAD assay (BIO RAD Laboratories, Richmond, California) and was found to be 5 to 7 mg cell protein per ml of lysate.

### Biosynthetic Assay Procedure

As described by Ielpi, L., Couso, R.O., and Dankert, M.A. in FEBS Letters 130:253-256 (1981), specifically incorporated herein by reference, an aliquot of freeze-thawed lysate (equivalent to 300 to 400 ug protein), DNAase I (10 ug/ml), and MgCl₂ (8 mM) were preincubated at 20°C for twenty minutes. An equal volume of 70 mM Tris-HCl, pH 8.2, with the desired radiolabeled sugar nucleotides (UDP-glucose, GDP-mannose and UDP-glucuronic acid) were added and incubated at 20°C. Radiolabeled phosphoenol pyruvate and acetyl coenzyme A were added when desired as described in Ielpi et al., supra, and Ielpi, L., Couso, R.O., and Dankert, M.A. Biochem. Biophys. Res. Comm. 102:1400-1408 (1981) and Ielpi, L., Couso, R.O., and Dankert, M.A., Biochem. Intern. 6:323-333 (1983), both of which are specifically incorporated herein by reference. At various times, the reactions were stopped by dilution with 4°C buffer. The samples were centrifuged and the pellets were washed two times with buffer. The supernatants were combined, carrier xanthan (100 ug) was added, and the xanthan plus synthesized polymer were precipitated with ethanol (60%)-KCl(0.8%). The precipitated polymer was resuspended in water and reprecipitated two more times to remove unincorporated label. Radioactivity incorporated into the precipitate (termed the gum fraction) was determined in a liquid scintillation counter and the data were processed to obtain incorporation in terms of picomoles of the radiolabeled components.

Cell lysates of X1006 did not incorporate carbon-14 acetate from [¹⁴C] acetyl CoA into the gum fraction of the in vitro system. Cell lysates of S4-L did produce in vitro gum radiolabeled with [¹⁴C] acetate. Similarly, cell lysates of X921 did not incorporate [¹⁴C] pyruvate into the gum fraction while S4-L cell lysates did incorporate radiolabeled pyruvate from phosphoenol [¹⁴C] pyruvate into the gum fraction of the in vitro system. Thus, X1006 was identified as a mutant strain with a defect in the gene for Acetylase and X921 as a mutant strain with a defect in the gene for Ketalase. Lysates of these strains produced non-acetylated xanthan and non-pyruvylated xanthan, respectively, in vitro.

It has also been shown that, by withholding substrates, X. campestris B1459 S4-L lysates produce altered polysaccharides in vitro. For example, cell lysates of S4-L did produce non-acetylated, non-pyruvylated xanthan gum in vitro when the endogenous acetyl-CoA and phosphoenolpyruvate were depleted.

### EXAMPLE 3

This example demonstrates the use of the Acetylase-deficient strain, X1006, to produce non-acetylated gum in vivo. This example also demonstrates the in vivo production of the non-pyruvylated gum from the Ketalase-minus strain, X921, and xanthan gum with a reduced level of pyruvylation from the strain X934.

The three mutant strains described above and S4-L were grown overnight in broth with two percent glucose at 30°C. The gum was harvested by removing cells by centrifugation, precipitating the gum from the supernatant by addition of 2-propanol or ethanol with 0.5 to 1% potassium chloride. The gum precipitate was recovered by centrifugation and resuspended. The procedure was repeated. The resuspended gum was dialysed against water. A sample of each polysaccharide was acid hydrolyzed and analyzed by HPLC using a BIO RAD HPX-87H column. Xanthan components were quantitated by the injection of standards of known concentration.

The HPLC analysis of the hydrolyzed gums showed that X921 produced xanthan gum without pyruvate. Strain X1006 produced xanthan gum with no acetate. Strain X934 produced a gum that contained pyruvate at a level of 1-5% that of S4-L gum.

### EXAMPLE 4

This example describes methods and strategies that could be employed to construct mutants of X. campestris that will produce xanthan that is both non-acetylated and non-pyruvylated.

Mutant strains of X. campestris lacking Ketalase (X921) or Acetylase (X1006) activity have been described. One could use microbial genetics and recombinant DNA methods as described by Vanderslice et al. and Capage et al. to introduce a Ketalase defect and an Acetylase defect into a single strain of X. campestris. This double-mutant strain would produce xanthan gum that was both non-acetylated and non-pyruvylated.

Methods for producing insertion mutations into cloned gum gene DNA carried on plasmid vectors have been described by Capage et al. One could envision using a plasmid carrying the insertion mutation that gave rise to mutant strain X921 as a substrate for a second round of mutagenesis in vivo or in vitro. This second round of mutagenesis could employ any of a number of transposable elements that would readily occur to one skilled in the art or any of an equally obvious number of DNA restriction fragments containing selectable markers. A set of plasmids carrying two insertions mutations could be used in the gene replacement technique of Capage et al. to transfer the plasmid-borne mutations into the chromosome of X. campestris. Phenotypes of the resultant strains can be analyzed by the in vivo and in vitro techniques described by Vanderslice et al. and Capage et al., supra, in Example 2. These analyses will reveal doubly mutant strains that are blocked in both the Ketalase and Acetylase activity. These double mutant strains will produce xanthan that is simultaneously non-pyruvylated and non-acetylated.

### EXAMPLE 5

This example discusses cloning the acetylase gene and the Ketalase gene onto vectors to ensure that the polysaccharides described herein are fully acetylated, fully pyruvylated, or both.

The X. campestris stains X1006 and X921 have mutations in the genes for acetylase and ketalase, respectively, as described in Examples 2 and 3. These mutants were created employing the methods described in Example 1. It is possible for one skilled in the art to employ methods described in Betlach et al. supra, to recover native DNA restriction fragments containing the Acetylase or Ketalase genes. That is, plasmids with DNA restriction fragments containing the Acetylase or Ketalase genes interrupted by a drug resistance marker can be used to probe lambda genomic libraries to obtain native DNA sequences for the Acetylase or Ketalase gene. In another embodiment, the genes for the acetylase and ketalase enzymes have already been cloned onto the plasmid pRK290-H336,
as described by Capage et al., supra, and other plasmids described therein. It is a simple matter for those skilled in the art to subclone the Acetylase and Ketalase genes themselves from these plasmids.

The native DNA sequences obtained by either method can then be inserted onto plasmids capable of replication in X. campestris, for instance pMW79, using the methods revealed in Betlach et al. and Capage et al. Expression of the Acetylase and/or Ketalase gene can be controlled by modifying the existing DNA sequence or inserting regulatable promoters upstream from the gene. Such techniques are well known to those versed in molecular biology and genetics. Similarly, the plasmids onto which the genes are inserted can be high copy number plasmids. As revealed in Capage et al., the xanthan biosynthetic enzymes are present in low amounts in X. campestris. Insertion of the plasmids described above into X. campestris, and growth of cultures under appropriate conditions for expression of the plasmid-borne genes, will result in synthesis of much greater numbers of the Acetylase and/or Ketalase enzymes than the other xanthan biosynthetic enzymes. Overexpression of the Acetylase should cause the xanthan polysaccharide to be fully acetylated, i.e., all internal mannose residues acetylated. Similarly, overexpression of the Ketalase should cause xanthan polysaccharide to be fully pyruvylated on the terminal mannose.

It should be evident to one skilled in the art that full acetylation and full pyruvylation of xanthan can be achieved by the methods described above.

### EXAMPLE 6

This example demonstrates the economic and technical advantages of a non-pyruvylated polysaccharide produced by a genetically modified Xanthomonas campestris for viscosifying water at high temperatures.

Xanthan gum, a natural product of Xanthomonas campestris, is an effective viscosifier of water for use in, for example, enhanced recovery of petroleum. These viscosity applications frequently necessitate xanthan gum to be applied at high temperature and in saline brines. Xanthan gums are effective viscosifiers even at high temperature (for example, 75° to 100°C), although their viscosity is substantially reduced over that at lower temperature (for example, 25° to 60°C).

The inventors have discovered a new and novel polysaccharide produced by a genetically modified strain of Xanthomonas campestris (strain X921) that produced a viscosity at high temperature equal to that of wild-type xanthan gum produced from the S4-L parent strain (strain X237). This xanthan gum has the normal pentameric xanthan structure but, because of the genetic modification, contains no pyruvate moiety on the terminal mannose. This novel polysaccharide has low viscosity at fermentation temperature near 30°C which will result in substantial cost savings and processing conveniences. The cost to produce pyruvylated xanthan gum is high primarily because the polymer's high viscosity requires great energy input for agitation, aeration and cooling.

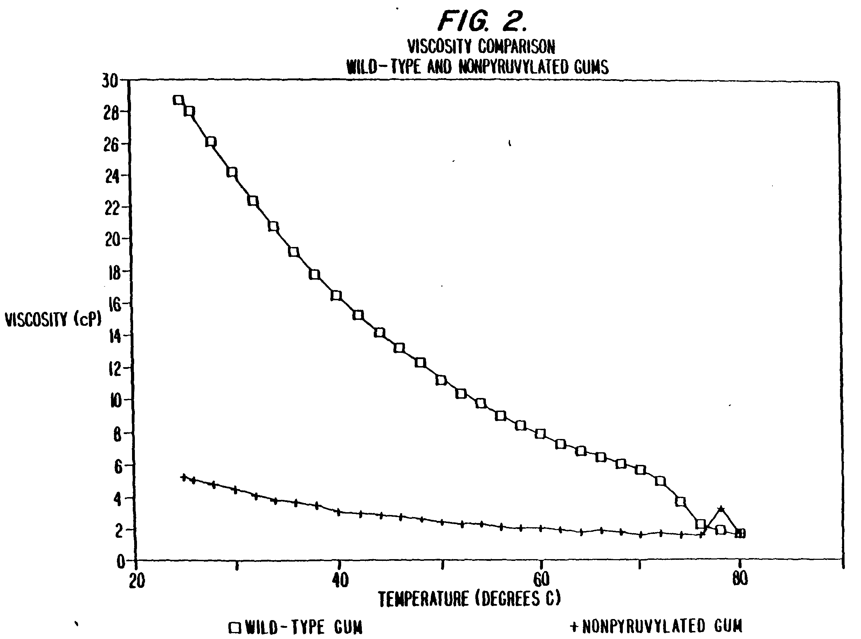

Figure 2 shows a viscosity comparison of the novel non-pyruvylated xanthan gum to a wild-type xanthan gum produced by the unmodified parent. The wild-type gum shows high viscosity at low temperature but the viscosity decreases rapidly with increasing temperature. The non-pyruvylated gum, on the other hand, has lower viscosity at low temperature, but retains a viscosity at high temperature essentially equivalent to the wild-type xanthan gum. The viscosities reported in Figure 2 were recorded at a shear rate of 8 s⁻¹ in a concentric cylinder viscometer on solutions of 1000 ppm active polymer solids in 5000 ppm NaCl brine.

### EXAMPLE 7

This example demonstrates the advantages of a non-acetylated xanthan produced by a genetically modified Xanthomonas campestris for use as a visosifying agent for aqueous solutions.

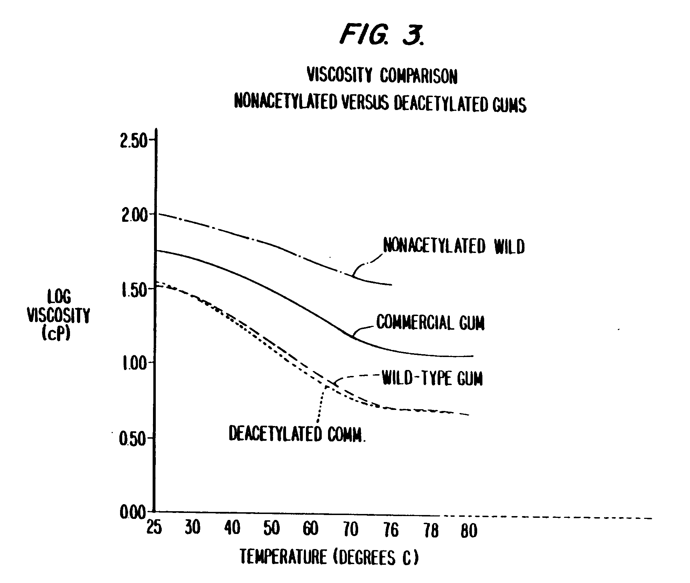

Figure 3 compares the viscosities of chemically deacetylated commercial xanthan and its parent compound with those of a non-acetylated xanthan polysaccharide made from a genetically manipulated Xanthomonas campestris (strain X1006) and of the xanthan gum made from the wild-type X. campestris parent (strain X237). The viscosities were obtained at a shear rate of 8 s⁻¹ for 1000 ppm polymer concentration in 50,000 ppm NaCl brine. Viscosities were measured over the temperature range of 25° to about 80°C.

Figure 3 demonstrates that chemical deacetylation of xanthan results in a loss of viscosifying power over the entire temperature range. However, elimination of acetylation by genetic means results in substantially increased viscosity compared to the wild-type xanthan gum. Thus, non-acetylated xanthan gum produced by a mutant strain of X. campestris is an improved polysaccharide compared to the xanthan itself and compared to deactetylated xanthan gum produced by chemical methods.

### Example 8

This example describes the methods used to construct a double mutuant strain of X. campestris that produces non-acetylated, non-pyruvylated xanthan.

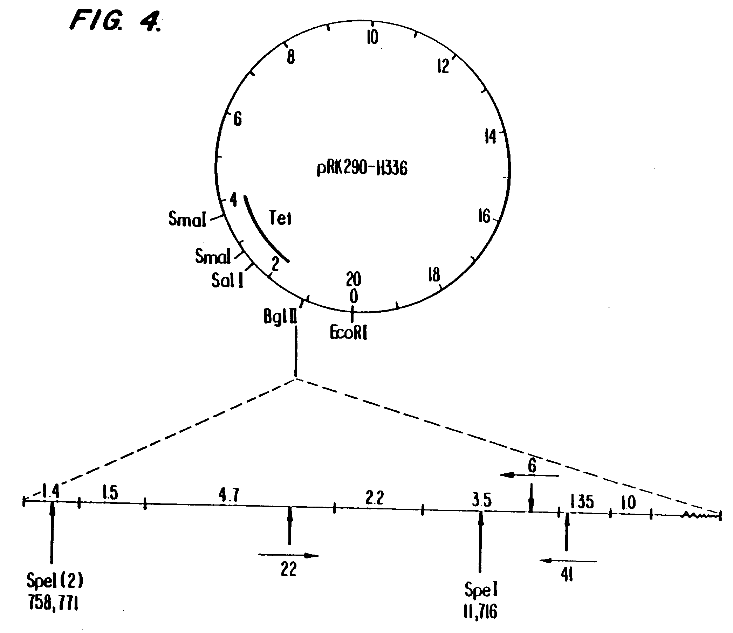

Capage et al. have described the cloning of a gene cluster from X. campestris that contains genes that direct the biosynthesis of xanthan gum. They also described the isolation of chromosomal deletion mutations in X. campestris that eliminate all or varying portions of this gene cluster. One such deletion mutant, strain X1231, lacks all of the X. campestris DNA that is carried on the recombinant plasmid pRK290-H336. Thus, strain X1231 does not synthesize xanthan. When pRK290-H336 is transferred into strain X1231, the ability to synthesize xanthan is restored. Capage et al. also described methods of isolating and characterizing insertion mutations of transposon TnK12 within the cloned gum gene DNA carried on pRK290-H336. Two such mutant plasmids are pRK290-H336.22 and pRK290-H336.41. The approximate locations of the TnK12 inserts on each of these plasmids is shown in Figure 4. When pRK290-H336.22 is transferred into X1231, the resultant strain produces non-acetylated xanthan. When pRK290-H336.41 is transferred into X1231, non-pyruvylated xanthan is produced. These two plasmid mutants have been used to construct, by means on in vitro recombination, a double mutant plasmid that directs synthesis of non-acetylated, non-pyruvylated xanthan when carried in the deletion strain X1231.

The in vitro strategy for generating a non-acetylated, non-pyruvylated double mutuant plasmid is as follows. A kanamycin-sensitive (Kan^{s}) derivative of pRK290-H336.41 was generated by doing a partial HindIII digestion of the plasmid, ligating the digestion products at very low DNA concentrations (to promote intramolecular ligation), and then screening tetracycline-resistant (Tet^{r}) transformants for kanamycin sensitivity. Plasmid DNAs were then prepared from Tet^{r}, Kan^{s} isolates and analyzed by restriction endonuclease digestion and agarose gel electrophoresis. There are only three HindIII sites in pRK290-H336.41, and two of these occur within TnK12 and bracket the Kan gene. Thus, a high proportion of the deletions generated in the partial digestion were deleted for the Kan gene whereas the rest of the plasmid was retained intact. The Kan^{s} plasmid still carries an insertion (1 kb) in the ketalase gene and thus was expected to yield non-pyruvylated gum. This plasmid is termed p41KS. The next step was to clone the large SpeI fragment of the non-acetylated mutant plasmid pRK290-H336.22 into p41KS. As shown in Figure 4, each plasmid contains three SpeI sites at positions 758, 771, and 11,716 within the DNA sequence of Capage et al. The 10.9 kb SpeI fragment carries the Tnk12 insertion of pRK290-H336.22. The small (13 bp) SpeI fragment lies entirely within a tRNA gene which which is nonessential for X. campestris growth and xanthan production. Thus, deletion of this small SpeI segment in the process of the double mutant construction ought not to affect xanthan biosynthesis. Plasmids p41KS and pRK290-H336.22 were purified and digested to completion with SpeI and a ligation was performed. In this ligation, p41KS/SpeI was ligated in 10x molar excess with H336.22/SpeI. Thus, when recombinants containing the Kan^{r} SpeI fragment of H336.22 were selected, they should most often be associated with the SpeI vector fragment of p41KS. We performed transformations with these ligations and obtained Kan^{r} transformants. The plasmids carried by these transformants were analyzed to identify the recombinants of interest. The desired recombinant plasmid was readily identified among the Kan^{r} transformants. This recombinant plasmid, termed p41KS22, contains the p41KS-derived insertion in the ketalase gene and the H336.22-derived TnK12 insertion within the acetylase gene. Appropriate restriction digestion analysis confirmed the presence of both insertion mutations and, furthermore, showed that the SpeI fragment containing the TnK12 mutation had been inserted in the correct orientation.

Plasmid p41KS22 was subsequently transferred into a series of X. campestris strains via conjugation. The large Gum⁻ deletion strain X1231 was among the recipients. This deletion lacks all of the gum gene DNA carried on p41KS22; therefore, X1231 carrying p41KS22 should produce non-acetylated, non-pyruvylated xanthan. The plasmid transferred efficiently into X1231, and the resultant phenotype was clearly mucoid but significantly less so than a wild-type control. Polysaccharide produced by X1231 carrying p41KS22 was prepared and analyzed. This polymer contained glucose, mannose, and glucuronic acid but no detectable acetate or pyruvate, demonstrating that X1231 (p41KS22) does produce the expected non-acetylated, non-pyruvylated gum.

### Example 9

This example describes the construction and properties of a double mutant plasmid that combines an Acetylase mutation and a Transferase IV mutation.

Capage et al. described methods for isolating and characterizing transposon TnK12 insetions within the cloned gum gene DNA carried by plasmid pRK290-H336. One mutant plasmid carrying such an insertion is pRK290-H336.6. The approximate location of the TnK12 insertion in this plasmid is shown in Figure 4. When present in the deletion strain X1231, this plasmid directs the synthesis of polytrimer gum as a result of the insertional inactivation of Transferase IV. Using procedures analogous to those described in Example 8, a double mutant plasmid was constructed that combines this Transferase IV defect with the Acetylase mutation carried in pRK290-H336.22. A kanamycin-sensitive derivative of pRK290-H336.6 was dervied by deletion of the HindIII fragment of TnK12. This plasmid, p6KS, is analogous to the Kan^{s} plasmid p41KS and still retains 1 kb of TnK12 inserted within the Transferase IV gene. Subsequently, the large TnK12-containing SpeI fragment of pRK290-H336.22 was ligated into SpeI-digested p6KS plasmid DNA as described in Example 8 and the double mutant plasmid p6KS22 was obtained. This plasmid carries insertion mutations in Transferase IV and the Acetylase. When transferred into deletion strain X1231, it ought to direct the synthesis on non-acetylated polytrimer. However, in several independent plasmid transfer experiments, no transfer of p6KS22 into strain X1231 was detected, although the plasmid was successfully transferred at high frequency into other recipients, including both Gum⁻ and Gum⁺ strains. This result suggests that the presence of p6KS22 in strain X1231 is lethal, probably as a direct result of production of non-acetylated polytrimer gum. Capage et al. described three other lethal mutations within the gum gene cluster and concluded that these lethal mutations cause the accumulation of a toxic intermediate in xanthan biosynthesis. Accumulation of non-acetylated polytrimer could potentially be toxic if this polysaccharide cannot be secreted by the transport system that normally secretes xanthan.

It is to be understood that application of the teachings of the present invention to a specific microorganism will be within capabilities of one having ordinary skill in the art in light of the teachings contained herein.

It will be apparent to those skilled in the art that various modifications and variations can be made in the processes and products of the present invention. Thus, it is intended that the present invention cover the modifications and variations of this invention provided they come within the scope of the appended claims.

## Claims

1. A process for preparing a water-soluble polysaccharide polymer comprising repeating pentamer units having a D-glucose: D-mannose: D-glucuronic acid ratio of about 2:2:1, wherein the D-glucose moieties are linked in a beta-[1,4] configuration, inner D-mannose moieties are linked in an alpha-[1,3] configuration primarily to alternate glucose moieties, the D-glucuronic acid moieties are linked in a beta-[1,2] configuration to said inner mannose moieties, and outer mannose moieties are linked to said glucuronic acid moieties in a beta-[1,4] configuration, wherein said polysaccharide polymer is not acetylated, said process comprising:
(a) obtaining an acetylase deficient mutant of Xanthomonas; and
b) culturing said Xanthomonas under conditions sufficient to produce said polysaccharide polymer.

2. The process of claim 1, wherein said polymer is not pyruvylated.

3. The process of claim 1, wherein said polymer is pyruvylated.

4. The process of claim 3, wherein said polymer is at least 90% pyruvylated.

5. The process of claim 4, wherein said Xanthomonas is Xanthomonas campestris.

6. The process of claim 5, wherein said Xanthomonas campestris is X1006 having accession number ATCC 53473.

7. The process of claim 1, wherein said Xanthomonas is Xanthomonas campestris.

8. The process of claim 2, wherein said Xanthomonas is a ketalase and acetylase deficient mutant of Xanthomonas.

9. The process of claim 8, wherein said Xanthomonas is Xanthomonas campestris.

10. The process of claim 9, wherein said Xanthomonas is Xanthomonas campestris X1231 having accession number ATCC 67344.

11. A mutant Xanthomonas capable of producing the polysaccharide polymer as defined in any one of claims 1 to 4.

12. The mutant Xanthomonas of claim 11, wherein said Xanthomonas is an acetylase deficient mutant of Xanthomonas.

13. The mutant Xanthomonas of claim 12, wherein said Xanthomonas is Xanthomonas campestris.

14. The mutant Xanthomonas capable of producing the polysaccharide polymer as defined in claim 2, wherein said Xanthomonas is a ketalase and acetylase deficient mutant of Xanthomonas.

15. The mutant Xanthomonas of claim 14, wherein said Xanthomonas is Xanthomonas campestris.

16. The mutant Xanthomonas of claim 15, wherein said Xanthomonas is Xanthomonas campestris X1231 having accession number ATCC 67344.

17. The mutant Xanthomonas X1006 having accession number ATCC 53473.

18. A water-soluble polysaccharide polymer comprising repeating pentamer units having a D-glucose:D-mannose:D-glucuronic acid ratio of about 2:2:1, wherein the D-glucose moieties are linked in a beta-[1,4] configuration, inner mannose moieties are linked in an alpha-[1,3] configuration primarily to alternate glucose moieties, the D-glucuronic acid moieties are linked in a beta-[1,2] configuration to said inner mannose moieties, and outer mannose moieties are linked to said glucuronic acid moieties in a beta-[1,4] configuration, wherein said polysaccharide polymer is not acetylated and has a viscosity greater than the viscosity of chemically deacetylated xanthan gum.

19. The polysaccharide polymer of claim 18, wherein said polymer has a viscosity greater than chemically deacetylated xanthan gum at temperatures from 25°C to 76°C.

20. The polysaccharide polymer of claim 19, wherein said polymer has a viscosity greater than chemically deacetylated xanthan gum at a temperature of 25°C.

21. The polysaccharide polymer of claim 20, wherein said polymer has a viscosity greater than chemically deacetylated xanthan gum at 25°C at a shear rate of 8s⁻¹ for 1000 ppm polymer concentration in 50,000 ppm NaCl brine.

22. The polysaccharide polymer of claim 21, wherein said polymer has a viscosity of about 2.0 cP.

23. The polysaccharide polymer of claim 21, wherein said polymer has a viscosity of greater than 1.5 cP at a temperature of 70°C.

24. A water-soluble polysaccharide polymer comprising repeating pentamer units having a D-glucose:D-mannose:D-glucuronic acid ratio of about 2:2:1, wherein the D-glucose moieties are linked in a beta-[1,4] configuration, inner mannose moieties are linked in an alpha-[1,3] configuration primarily to alternate glucose moieties, the D-glucuronic acid moieties are linked in a beta-[1,2] configuration to said inner mannose moieties, and outer mannose moieties are linked to said glucuronic acid moieities in a beta-[1,4] configuration, wherein said polysaccharide polymer is not acetylated and has a viscosity greater than the viscosity of xanthan gum.

## Patentansprüche

1. Verfahren zur Herstellung eines wasserlöslichen Polysaccharid-Polymers, umfassend sich wiederholende pentamere Einheiten mit einem D-Glucose: D-Mannose: D-Glucuronsäureverhältnis von ungefähr 2:2:1, worin die D-Glucoseanteile in beta-[1,4]-Konfiguration verknüpft sind, innere D-Mannoseanteile in einer alpha-[1,3]-Konfiguration im wesentlichen an alternierende Glucoseanteile gebunden sind, die D-Glucuronsäureanteile in einer beta[1,2]-Konfiguration an die inneren Mannoseanteile gebunden und äußere Mannoseanteile an die Glucuronsäureanteile in einer beta-[1,4]-Konfiguration gebunden sind, wobei das Polysaccharid-Polymer nicht acetyliert ist, wobei das Verfahren folgende Schritte umfaßt:
(a) Erhalten eines Acetylase-defizienten Mutanten von Xanthomonas; und
(b) Kultivierung des Xanthomonas unter Bedingungen, die geeignet sind, das Polysaccharid-Polymer zu produzieren.

2. Verfahren gemäß Anspruch 1, wobei das Polymer nicht pyruvyliert ist.

3. Verfahren gemäß Anspruch 1, wobei das Polymer pyruvyliert ist.

4. Verfahren gemäß Anspruch 3, wobei das Polymer zu mindestens 90% pyruvyliert ist.

5. Verfahren gemäß Anspruch 4, wobei die Xanthomonas-Art Xanthomonas campestris ist.

6. Verfahren gemäß Anspruch 5, wobei Xanthomonas campestris X1006 mit der Hinterlegungsnummer ATCC 53473 ist.

7. Verfahren gemäß Anspruch 1, wobei die Xanthomonas-Art Xanthomonas campestris ist.

8. Verfahren gemäß Anspruch 2, wobei Xanthomonas ein Ketalase- und Acetylase-defizienter Mutant von Xanthomonas ist.

9. Verfahren gemäß Anspruch 8, wobei die Xanthomonas-Art Xanthomonas campestris ist.

10. Verfahren gemäß Anspruch 9, wobei die Xanthomonas-Art Xanthomonas campestris X1231 mit der Hinterlegungsnummer ATCC 67344 ist.

11. Ein mutanter Xanthomonas, der fähig ist zur Produktion des Polysaccharid-Polymers, definiert wie in einem oder mehreren Ansprüchen 1 bis 4.

12. Mutanter Xanthomonas gemäß Anspruch 11, wobei der Xanthomonas ein Acetylase-defizienter Mutant von Xanthomonas ist.

13. Mutanter Xanthomonas gemäß Anspruch 12, wobei der Xanthomonas Xanthomonas campestris ist.

14. Mutanter Xanthomonas, der fähig ist zur Produktion des Polysaccharid-Polymers, wie definiert in Anspruch 2, wobei der Xanthomonas ein Ketalase- und Acetylase-defizienter Mutant von Xanthomonas ist.

15. Mutanter Xanthomonas gemäß Anspruch 14, wobei der Xanthomonas Xanthomonas campestris ist.

16. Mutanter Xanthomonas gemäß Anspruch 15, wobei der Xanthomonas Xanthomonas campestris X1231 mit der Hinterlegungsnummer ATCC 67344 ist.

17. Mutanter Xanthomonas X1006 mit der Hinterlegungsnummer ATCC 53473.

18. Ein wasserlösliches Polysaccharid-Polymer, umfassend sich wiederholende pentamere Einheiten, mit einem D-Glucose: D-Mannose: D-Glucuronsäureverhältnis von ungefähr 2:2:1, wobei die D-Glucoseanteile in einer beta-[1,4]-Konfiguration verknüpft sind, innere Mannoseanteile in einer alpha[1,3]-Konfiguration im wesentlichen an alternierende Glucoseanteile gebunden sind, die D-Glucuronsäureanteile in einer beta-[1,2]-Konfiguration an die inneren Mannoseanteile gebunden sind und äußere Mannoseanteile mit den Glucuronsäureanteilen in einer beta-[1,4]-Konfiguration verbunden sind, wobei das Polysaccharid-Polymer nicht acetyliert ist, und eine Viskosität aufweist, die größer ist als die Viskosität von chemisch deacetyliertem Xanthan-Gummi.

19. Polysaccharid-Polymer gemäß Anspruch 18, wobei das Polymer eine Viskosität aufweist, die größer ist als die von chemisch deacetyliertem Xanthan-Gummi bei Temperaturen von 25°C bis 76°C.

20. Polysaccharid-Polymer gemäß Anspruch 19, wobei das Polymer eine Viskosität aufweist, die größer ist als die von chemisch deacetyliertem Xanthan-Gummi bei einer Temperatur von 25°C.

21. Polysaccharid-Polymer gemäß Anspruch 20, wobei das Polymer eine Viskosität aufweist, die größer ist als die von chemisch deacetyliertem Xanthan-Gummi bei 25°C bei einer Scherungsrate von 8s⁻¹ für eine 1000 ppm Polymerkonzentration in 50.000 ppm NaCl-Sole.

22. Polysaccharid-Polymer gemäß Anspruch 21, wobei das Polymer eine Viskosität von ungefähr 2,0 cP aufweist.

23. Polysaccharid-Polymer gemäß Anspruch 21, wobei das Polymer eine Viskosität aufweist, die größer ist als 1,5 cP bei einer Temperatur von 70°C.

24. Wasserlösliches Polysaccharid-Polymer, umfassend sich wiederholende pentamere Einheiten, mit einem D-Glucose: D-Mannose: D-Glucuronsäureverhältnis von ungefähr 2:2:1, wobei die D-Glucoseanteile in beta-[1,4]-Konfiguration verknüpft sind, innere Mannoseanteile in einer alpha-[1,3]-Konfiguration im wesentlichen an alternierende Glucoseanteile gebunden sind, die D-Glucuronsäureanteile in einer beta-[1,2]-Konfiguration an die inneren Mannoseanteile gebunden sind und äußere Mannoseanteile an die Glucuronsäureanteile in einer beta-[1,4]-Konfiguration gebunden sind, wobei das Polysaccharid-Polymer nicht acetyliert ist und eine Viskosität aufweist, die größer ist als die Viskosität von Xanthan-Gummi.

## Revendications

1. Procédé pour préparer un polymère polysaccharide soluble dans l'eau comprenant des motifs récurrents de pentamère ayant un rapport D-glucose/D-mannose/acide D-glucuronique d'environ 2/2/1, dans lequel les portions de D-glucose sont liées dans une configuration β-[1,4], des portions internes de D-mannose sont liées dans une configuration α-[1,3] essentiellement toutes les deux portions de glucose, les portions d'acide D-glucuronique sont liées dans une configuration β-[1,2] auxdites portions internes de mannose, et des portions externes de mannose sont liées auxdites portions d'acide glucuronique dans une configuration β-[1,4], dans lequel ledit polymère polysaccharide n'est pas acétylé, ledit procédé comprenant :
(a) l'obtention d'un mutant déficient en acétylase de Xanthomonas; et
(b) la culture dudit Xanthomonas dans des conditions suffisantes pour produire ledit polymère polysaccharide.

2. Procédé selon la revendication 1, dans lequel ledit polymère n'est pas pyruvylé.

3. Procédé selon la revendication 1, dans lequel ledit polymère est pyruvylé.

4. Procédé selon la revendication 3, dans lequel ledit polymère est pyruvylé à 90% au moins.

5. Procédé selon la revendication 4, dans lequel ledit Xanthomonas est Xanthomonas campestris.

6. Procédé selon la revendication 5, dans lequel ledit Xanthomonas campestris est X1006 ayant le numéro d'acceptation à l'ATCC 53473.

7. Procédé selon la revendication 1, dans lequel ledit Xanthomonas est Xanthomonas campestris.

8. Procédé selon la revendication 2, dans lequel ledit Xanthomonas est un mutant déficient en kétalase et en acétylase de Xanthomonas.

9. Procédé selon la revendication 8, dans lequel ledit Xanthomonas est Xanthomonas campestris.

10. Procédé selon la revendication 9, dans lequel ledit Xanthomonas est Xanthomonas campestris X1231 ayant le numéro d'acceptation à l'ATCC 67344.

11. Xanthomonas mutant capable de produire le polymère polysaccharide tel que défini à l'une quelconque des revendications 1 à 4.

12. Xanthomonas mutant selon la revendication 11, dans lequel ledit Xanthomonas est un mutant déficient en acétylase de Xanthomonas.

13. Xanthomonas mutant selon la revendication 12, dans lequel ledit Xanthomonas est Xanthcmonas campestris.

14. Xanthomonas mutant capable de produire le polymère polysaccharide tel que défini à la revendication 2, dans lequel ledit Xanthomonas est un mutant déficient en kétalase et en acétylase de Xanthomonas.

15. Xanthomonas mutant selon la revendication 14, dans lequel ledit Xanthomonas est Xanthomonas campestris.

16. Xanthomonas mutant selon la revendication 15, dans lequel ledit Xanthomonas est Xanthomonas campestris X1231 ayant le numéro d'acceptation à l'ATCC 67344.

17. Xanthomonas mutant X1006 ayant le numéro d'acceptation à l'ATCC 53473.

18. Polymère polysaccharide soluble dans l'eau comprenant des motifs récurrents de pentamère ayant un rapport D-glucose/D-mannose/acide D-glucuronique d'environ 2/2/1, dans lequel les portions de D-glucose sont liées dans une configuration β-[1,4], des portions internes de mannose sont liées dans une configuration α-[1,3] essentiellement toutes les deux portions de glucose, les portions d'acide D-glucuronique sont liées dans une configuration β-[1,2] auxdites portions internes de mannose, et des portions externes de mannose sont liées auxdites portions d'acide glucuronique dans une configuration β-[1,4], dans lequel ledit polymère polysaccharide n'est pas acétylé et a une viscosité supérieure à la viscosité de la gomme de xanthane chimiquement désacétylée.

19. Polymère polysaccharide selon la revendication 18, dans lequel ledit polymère a une viscosité supérieure à celle de la gomme de xanthane chimiquement désacétylée à des températures de 25°C à 76°C.

20. Polymère polysaccharide selon la revendication 19, dans lequel ledit polymère a une viscosité supérieure à celle de la gomme de xanthane chimiquement désacétylée à une température de 25°C.

21. Polymère polysaccharide selon la revendication 20, dans lequel ledit polymère a une viscosité supérieure à celle de la gomme de xanthane chimiquement désacétylée à 25°C à un gradient de cisaillement de 8 s⁻¹ pour une concentration de polymère de 1000 ppm dans 50 000 ppm de saumure de NaCl.

22. Polymère polysaccharide selon la revendication 21, dans lequel ledit polymère a une viscosité d'environ 2,0 cP.

23. Polymère polysaccharide selon la revendication 21, dans lequel ledit polymère a une viscosité supérieure à 1,5 cP à une température de 70°C.

24. Polymère polysaccharide soluble dans l'eau comprenant des motifs récurrents de pentamère ayant un rapport D-glucose/D-mannose/acide D-glucuronique d'environ 2/2/1, dans lequel les portions de D-glucose sont liées dans une configuration β-[1,4], des portions internes de mannose sont liées dans une configuration α-[1,3] essentiellement toutes les deux portions de glucose, les portions d'acide D-glucuronique sont liées dans une configuration β-[1,2] auxdites portions internes de mannose, et des portions externes de mannose sont liées auxdites portions d'acide glucuronique dans une configuration β-[1,4], dans lequel ledit polymère polysaccharide n'est pas acétylé et a une viscosité supérieure à la viscosité de la gomme de xanthane.
